(19) Europäisches Patentamt
European Patent Office
Office européen des brevets

(11) Veröffentlichungsnummer: **0 256 532 B1**

(12) **EUROPÄISCHE PATENTSCHRIFT**

(45) Veröffentlichungstag der Patentschrift: **20.05.92**

(51) Int. Cl.5: **A61K 31/35**, C07D 311/66

(21) Anmeldenummer: **87111781.8**

(22) Anmeldetag: **13.08.87**

(54) **Verwendung von Dihydrobenzopyranderivaten.**

(30) Priorität: **15.08.86 US 897450**

(43) Veröffentlichungstag der Anmeldung:
**24.02.88 Patentblatt 88/08**

(45) Bekanntmachung des Hinweises auf die Patenterteilung:
**20.05.92 Patentblatt 92/21**

(84) Benannte Vertragsstaaten:
**BE CH DE FR GB IT LI LU NL SE**

(56) Entgegenhaltungen:
**EP-A- 0 129 906**
**EP-A- 0 139 809**
**EP-A- 0 150 447**
**EP-A- 0 156 233**

(73) Patentinhaber: **F. HOFFMANN-LA ROCHE AG**
**Postfach 3255**
**CH-4002 Basel(CH)**

(72) Erfinder: **Gaginella, Timothy Samuel**
**8 Lake Drive East**
**Wayne, N.J. 07470(US)**
Erfinder: **Welton, Ann Frances**
**37 Maple Drive**
**North Caldwell, N.J. 07006(US)**
Erfinder: **Will, Peter Graig**
**14J River Road**
**Nutley, N.J. 07110(US)**

(74) Vertreter: **Lederer, Franz, Dr. et al**
**Patentanwalt Dr. Franz Lederer Lucile-Grahn-Strasse 22**
**W-8000 München 80(DE)**

EP 0 256 532 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft die Verwendung einer Verbindung der allgemeinen Formel

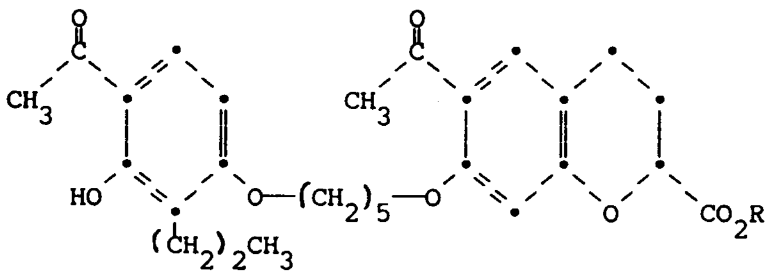

I

worin R Wasserstoff oder niederes Alkyl bedeutet,
eines Enantiomeren davon oder, falls R Wasserstoff bedeutet, eines pharmazeutisch annehmbaren Salzes davon mit einer Base zur Herstellung von pharmazeutischen Präparaten gegen Enteritis und anderen durch Leukotrien vermittelten Entzündungen der intestinalen Mucosa.

Die Verbindungen der Formel I können auf verschiedenen Wegen, vorzugsweise oral, aber auch rektal oder parenteral, verabreicht werden. Die pharmazeutischen Präparate, welche als Wirkstoff eine der obigen Verbindungen enthalten, liegen als orale Dosierungsformen, zwecks Freigabe des Wirkstoffes im Dünn- und im Dickdarm, oder als rektale oder parenterale Dosierungsformen vor.

Die obigen Verbindungen und Präparate können verwendet werden, um Schleimhautentzündungen des Darmes einschliesslich der Enteritis, welche, wie es scheint, durch Leukotrien vermittelt werden, wirkungsvoll zu behandeln.

Der Ausdruck "Enteritis" wird häufig zur Bezeichnung von Entzündungen des Darms oder anderer Teile des Intestinaltraktes verwendet, welche als gemeinsames Merkmal eine chronische oder akute Entzündung der gastrointestinalen Schleimhäute aufweisen. Verschiedene Formen schliessen im allgemeinen die Crohn-Krankheit des Ileums und des Kolons, die Colitis ulcerosa, die neonatale nekrotisierende Enterokolitis und Lebensmittelallergien ein. Histopathologisch sind sie durch Ulceration, Pseudomembrane, radiologisch sichtbare Läsionen, Oedeme und durch die Bildung von Entzündungszellen charakterisiert und haben als Symptome Diarrhö, abdominale Schmerzen, Gewichtsverlust und Hypoproteinämie zur Folge. Beschreibungen finden sich in den folgenden Literaturstellen: Northfield, Drugs 14: 198-206 (1977); Blaker et al., Eur. J. Pediatr. 139: 162-164 (1982); Singleton, The Gastroenterology Annual, S. 268-310 (1983); Saco et al., J. Amer. Acad. Dermatol. 4: 619-629 (1981); Prantera et al., Ital. J. Gastroenterol. 13: 24-27 (1981); Sales et al., Arch. Int. Med. 143: 294-299 (1983), und Ament, Inflammatory Bowel Diseases, Martinus Nijhoff Publ., Boston, Mass., S. 254-268 (1982). Weniger häufig aber auch möglich sind Schleimhautentzündungen anderer Teile des gastrointestinalen Traktes, wie die Duodenitis, die Jejunitis und die Proctitis.

Unter den Wirkstoffen, welche für die Behandlung derartiger Krankheiten nützlich sind, seien Sulfasalazin und andere Verbindungen, welche 5-Aminosalicylat im Darm freisetzen, Corticosteroide, Metronidazol und Cholestyramin erwähnt, vgl. Sack and Peppercorn in Pharmacotherapy 3, 158-176 (1983).

Die Verbindungen der Formel I, ihre Enantiomeren und ihre pharmazeutisch annehmbaren Salze sind bereits früher für andere Zwecke in der Europäischen Patentpublikation Nr. 129 906 beschrieben worden, und zwar insbesondere deren Verwendung als Antiallergika einschliesslich ihrer Verwendung zur Behandlung von Asthma. Andere Dihydrobenzopyranderivate sind in den europäischen Patentpublikationen 139809 (8. Mai 1985) und 150447 (7. Juni 1985), als Leukotrien-Inhibitoren beschrieben worden, welche nützlich sind zur Behandlung von Allergien sowie entzündlichen Erkrankungen, wie die rheumatoide Arthritis. In der Europäischen Patentpublikation Nr. 156 233 wird die Verwendung von strukturell unterschiedlichen Benzopyranderivaten (Leukotrien-Antagonisten) zur Herstellung cytoprotektiver pharmazeutischer Präparate zur Behandlung erosiver Gastritis, Oesophagitis und entzündlicher Darmkrankheiten beschrieben. Die vorliegende Erfindung beruht auf der Erkenntnis, dass die hier spezifisch beschriebenen Verbindungen bei der Bekämpfung der oben erwähnten Schleimhautentzündungen ganz besonders nützlich und ausserordentlich potent sind.

Der Ausdruck "Leukotrien-vermittelt" wird verwendet, um anzudeuten, dass die Substanz Leukotrien an der Verursachung der entzündlichen Hauterkrankung oder an der Manifestation der damit verbundenen Symptome oder an beidem aktiv teilnimmt.

Der Ausdruck "niederes Alkyl" bezeichnet im Rahmen der vorliegenden Erfindung geradkettige oder verzweigte gesättigte Kohlenwasserstoffreste mit 1-7 Kohlenstoffatomen, beispielsweise Methyl, Aethyl, Propyl, Isopropyl, Butyl, t-Butyl, Neopentyl, Pentyl, Hexyl und Heptyl.

Die in der vorliegenden Beschreibung verwendeten Ausdrücke "HAL" und "Halogen" bezeichnen die vier Halogene Brom, Chlor, Fluor und Jod, und zwar vorzugsweise Brom, Chlor und Jod.

Die Verbindungen der Formel I können wie nachfolgend beschrieben gemäss Reaktionsschema I hergestellt werden:

**Reaktionsschema I**

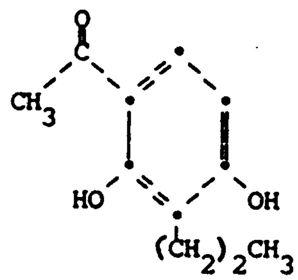

+

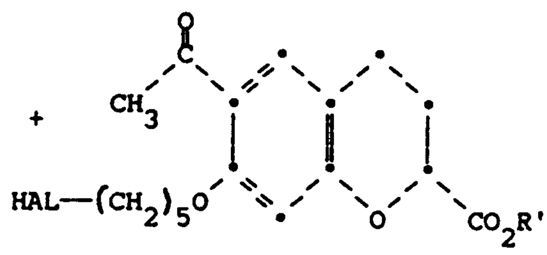

II III

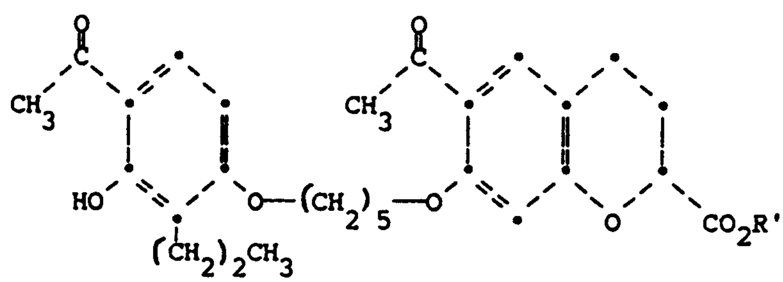

Ia

(fakultativ)

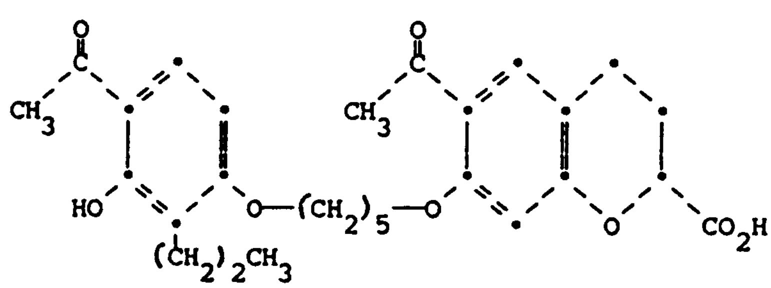

Ib

worin R′ niederes Alkyl bedeutet, und HAL die obige Bedeutung besitzt.

Die Umsetzung der Verbindung der Formel II (einer bekannten Verbindung) mit einer Verbindung der Formel III gemäss Reaktionsschema I zu einer Verbindung der Formel Ia wird unter wasserfreien Bedingungen in einem inerten Lösungsmittel, beispielsweise in Aceton, Acetonitril, Methyläthylketon, Diäthylketon, Dimethylformamid oder dergleichen, durchgeführt. Die Reaktion wird bei der Rückflusstemperatur des

Reaktionsgemisches, vorzugsweise bei einer Temperatur im Bereich von etwa 70 ° C bis etwa 100 ° C, und in Gegenwart eines säurebindenden Mittels, wie Kaliumcarbonat oder dergleichen, durchgeführt. Als Lösungsmittel verwendet man vorzugsweise ein Gemisch von Aceton und Dimethylformamid. Die erhaltene Verbindung der Formel Ia kann mittels herkömmlicher Methoden, beispielsweise durch Kristallisation, chromatographische Methoden oder dergleichen, isoliert werden.

Die Verbindung der Formel Ia, welche selber erfindungsgemäss verwendet werden kann, kann erwünschtenfalls durch Hydrolyse in eine Verbindung der Formel Ib übergeführt werden. Die Hydrolyse wird mit einem Alkalimetallhydroxid, beispielsweise Lithiumhydroxid, Natriumhydroxid, Kaliumhydroxid oder dergleichen, in einem Gemisch aus Wasser und einem mit Wasser mischbaren Lösungsmittel, beispielsweise Methanol, Aethanol, Tetrahydrofuran oder dergleichen, bei einer Temperatur im Bereich von etwa Raumtemperatur (beispielsweise 20 ° bis 25 ° C) bis zur Rückflusstemperatur durchgeführt. Die erhaltene Verbindung der Formel Ib kann mittels herkömmlicher Methoden, beispielsweise durch Extraktion, Kristallisation, chromatogrpahische Methoden oder dergleichen, isoliert werden.

Die erfindungsgemässe Verwendung kann auch mit pharmazeutisch annehmbaren Salzen von Verbindungen der Formel I, worin R Wasserstoff bedeutet, durchgeführt werden. Diese Salze können hergestellt werden, indem man eine Säure der Formel I oder ein Enantiomer davon mit einer Base umsetzt, welche ein nicht-toxisches und pharmakologisch und pharmazeutisch annehmbares Kation besitzt. Ganz allgemein umfasst die vorliegende Erfindung Salze mit sämtlichen Basen, welche mit den vorliegenden Carbonsäuren ein Salz bilden und deren pharmakologische Eigenschaften nicht zu nachteiligen physiologischen Reaktionen führen, wenn sie durch einen Warmblüter eingenommen werden. Geeignete Basen sind beispielsweise Alkalimetall- und Erdalkalimetallhydroxide und -carbonate, wie Natriumhydroxid, Kaliumhydroxid, Calciumhydroxid, Kaliumcarbonat und dergleichen, Ammoniak, primäre, sekundäre und tertiäre Amine, wie Monoalkylamine, Dialkylamine, Trialkylamine, stickstoffhaltige heterocyclische Amine, z.B. Piperidin, basische Aminosäuren, wie Lysin, und dergleichen. Die so erhaltenen pharmazeutisch annehmbaren Salze sind funktionelle Aequivalente der entsprechenden 3,4-Dihydro-2H-1-benzopyrancarbonsäure der Formel Ib und dessen Enantiomeren. Dem Fachmann bereitet die Erkennung des Umfanges der therapeutisch verwertbaren Salze keine Schwierigkeiten, da die Vielfalt der erfindungsgemäss verwendbaren Salze nur durch die Kriterien beschränkt wird, dass die für die Herstellung dieser Salze verwendeten Basen nicht-toxisch und physiologisch annehmbar sind.

Die für die Herstellung von Verbindungen der Formel I verwendeten Ausgangsstoffe der Formel III sind bekannt oder können, wie nachfolgend beschrieben, gemäss dem Reaktionsschema II hergestellt werden:

## Reaktionsschema II

$HAL-(CH_2)_5-HAL$ +

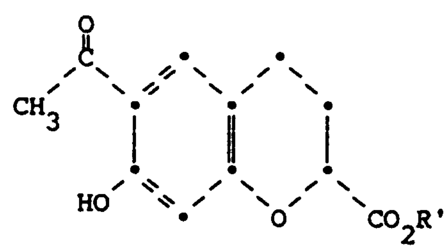

IV

V

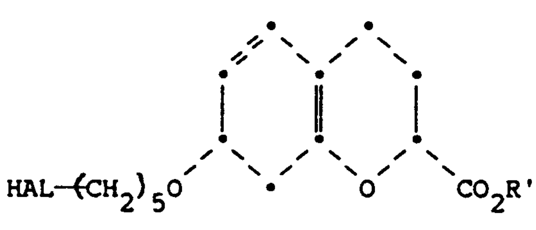

III

worin HAL und R′ obige Bedeutung besitzen.

Die Umsetzung einer Verbindung der Formel IV, mit einer Verbindung der Formel V gemäss Reaktionsschema II zu einer Verbindung der Formel III wird in einem inerten organischen Lösungsmittel, wie Dimethylformamid, Aceton, Methyläthylketon, Acetonitril oder dergleichen, vorzugsweise in Acetonitril, durchgeführt. Die Reaktion wird in Gegenwart einer Base, beispielsweise einem Alkalimetallcarbonat, wie Kaliumcarbonat, Natriumcarbonat oder dergleichen, oder einem Alkalimetallhydrid, wie Natriumhydrid oder dergleichen, bei einer Temperatur im Bereich von etwa 20° bis etwa 150° C, vorzugsweise bei Raumtemperatur (z.B. 20° bis 25° C) durchgeführt. Die erhaltene Verbindung der Formel III kann mittels herkömmlicher Methoden, beispielsweise mittels Kristallisation, Extraktion, Chromatographie oder dergleichen, isoliert werden.

Die als Ausgangsstoffe verwendeten Verbindungen der Formel V sind bekannt oder können nach an sich bekannten Methoden hergestellt werden.

Die für die Herstellung von Verbindungen der Formel III verwendeten Ausgangsstoffe der Formel V sind bekannt oder können, wie nachfolgen beschrieben, gemäss dem Reaktionsschema III hergestellt werden:

Reaktionsschema III

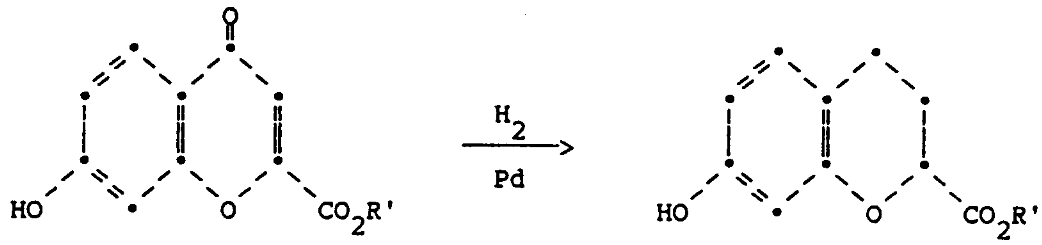


VI Va

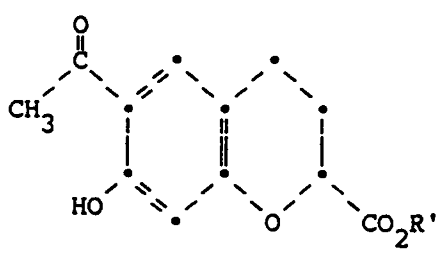

V

worin R′ obige Bedeutung besitzt.

Gemäss Reaktionsschema III wird eine Verbindung der Formel VI, welche bekannt ist oder nach an sich bekannten Methoden hergestellt werden kann, zu einer Verbindung der Formel Va hydriert. Im speziellen wird die Reaktion in Gegenwart eines Katalysators, beispielsweise Palladium auf Kohle, in einem Lösungsmittel, wie den niederen Fettsäuren, vorzugsweise in Essigsäure, bei einer Temperatur im Bereich von etwa 20° C bis etwa 100° C, vorzugsweise bei 25° C und bei erhöhtem Druck, vorzugsweise bei 50 psi (344,75 kPa) durchgeführt. Die erhaltene Verbindung der Formel Va kann mittels herkömmlicher Methoden isoliert werden.

Die Verbindung der Formel Va wird anschliessend mittels eines geeigneten Acetylierungsmittels, beispielsweise mittels eines Essigsäurehalogenides oder -anhydrides, vorzugsweise Essigsäureanhydrid, in einer geeigneten Base, beispielsweise in einem niederen Alkylamin oder dergleichen, vorzugsweise in Pyridin, bei einer Temperatur im Bereich von etwa 0° C bis etwa 150° C, vorzugsweise bei 25° C, in das entsprechende Acetylderivat der Formel V übergeführt. Die erhaltene Verbindung der Formel V kann mittels herkömmlicher Methoden isoliert werden.

Die Verbindungen der Formel I besitzen ein asymmetrisch substituiertes Kohlenstoffatom und fallen daher normalerweise als racemische Gemische an. Die Spaltung derartiger Racemate in die optisch aktiven Isomeren kann nach an sich bekannten Methoden durchgeführt werden. Gewisse racemische Mischungen können als Eutektika ausgefällt und anschliessend aufgetrennt werden. Vorzugsweise wird die Aufspaltung

jedoch auf chemischem Wege bewerkstelligt. In diesem Fall werden aus dem racemischen Gemisch von Verbindungen der Formel I, worin R Wasserstoff bedeutet, mit einem optisch aktiven Spaltungsmittel die entsprechenden Diastereoisomeren hergestellt. Geeignete Spaltungsmittel sind beispielsweise optisch aktive Basen, wie d-(+)- oder 1-(-)-α-Methylbenzylamin.Die erhaltenen diastereomeren Salze werden durch fraktionierte Kristallisation getrennt und anschliessend in die entsprechenden optischen Isomeren übergeführt. Die vorliegende Erfindung umfasst somit die Verwendung von Racematen von Verbindungen der Formel I als auch ihrer optisch aktiven Isomeren (Enantiomeren).

Weitere Details und spezifischere Methoden für die Herstellung von Verbindungen der Formel I können der weiter oben erwähnten Europäischen Patentpublikation Nr. 129 906 entnommen werden.

Wegen der Natur der zu behandelnden Krankheit werden die Verbindungen der Formel I vorzugsweise oral verabreicht, was dazu führt, dass die Oberfläche der entzündeten intestinalen Gewebe direkt der Wirkung der verabreichten Verbindung ausgesetzt werden. Für die orale Anwendung können diese Verbindungen in Form von Tabletten, Pillen, Kapseln, Pulvern, Granulaten oder Mikrokapseln verabreicht werden. Geeignete inerte pharmazeutisch annehmbare Träger für derartige Arzneiformen sind beispielsweise Talk, Stärke, Milchzucker und dergleichen. Sie können aber auch in Form von wässrigen Lösungen, Suspensionen, Elixieren oder alkoholischen Lösungen verabreicht werden. Sie können beispielsweise noch Aromastoffe, Färbemittel, Verdickungsmittel oder andere herkömmliche pharmazeutische Hilfsstoffe enthalten. In einer typischen Ausführungsform werden die Verbindungen der Formel I zu Präparaten formuliert, welche den Wirkstoff verlangsamt und insbesondere in den unteren Teilen des Darmes abgeben.

Andererseits können die Verbindungen der Formel I auch parenteral oder rektal mittels entsprechender Dosierungsformen verabreicht werden, welche geeignete Trägermaterialien enthalten. An sich bekannte und jedem Fachmann geläufige Herstellungsmethoden können zu deren Herstellung verwendet werden.

Im Sinne einer detaillierteren Beschreibung enthält der experimentelle Teil der vorliegenden Beschreibung auch Beispiele für erfindungsgemäss verwendbare oral, rektal und parenteral verabreichbare Dosierungsformen.

Die Dosierung von Verbindungen der Formel I und die Häufigkeit der Verabreichung hängen ab von der Stärke der Entzündung, vom Alter des zu behandelnden Patienten, sowie von der Potenz, der Wirkungsdauer und der Konzentration der speziellen Verbindung, welche als Wirkstoff verwendet wird.

Im Rahmen der erfindungsgemässen Verwendung der Verbindungen der Formel I ist mit Dosierungen im Bereich von etwa 50 bis 4000 mg/Tag, vorzugsweise von etwa 150 bis etwa 500 mg/Tag zu rechnen, wobei diese als Einzeldosierung oder in mehreren Dosen täglich verabreicht werden können.

Die wertvollen, erfindungsgemässen entzündungshemmenden Eigenschaften können anhand von pharmakologischen Standardverfahren in Warmblütern ermittelt werden. Die nachfolgend beschriebenen Methoden sind Beispiele solcher Standardmethoden:

A: Versuch mit der Mucosa des Rattenkolons, in vitro:

Das Bioassay-System des Versuches mit der Mucosa des Rattenkolons basiert auf der Messung der durch Absorption oder Sekretion bewirkten Aenderungen im Salztransport durch das tierische Gewebe, wobei man die durch Ussing et al. beschriebene Standard-Kurzschluss-Technik verwendet, Acta Physiol. Scand. 23, 110-127 (1951). Diarrhö ist das häufigste Symptom bei gastrointestinalen Entzündungen und tritt bei allen Typen der Enteritis auf. Pantera, et al., Ital. J. Gastroenterol. 13: 24-27 (1981); Sales, et al., Arch. Int. Med. 143: 294-299 (1983); Hodgson, Br. J. Clin. Pharmac. 14: 159-270 (1982). Diarrhö wird durch eine Abnahme in der Salzabsorption oder eine Zunahme in der Salzsekretion im Darm bewirkt und kann durch die oben erwähnte Methode von Ussing et al. in vitro quantifiziert werden. Man entfernt von 200-250 g schweren Tieren ein 1 cm langes Segment des Kolons, behandelt dieses in vitro mit Leukotrienen oder Bradykinin, welche die Salzsekretion fördern, und misst die erhaltene Aenderung im Netto-Salztransport, welche in Gegenwart und in Abwesenheit von 6-Acetyl-7-[5-(4-acetyl-3-hydroxy-2-propylphenoxy)-pentyloxy] -3,4-dihydro-2H-1-benzopyran-2-carbonsäure als Hemmer erhalten wird. Gewebeproben werden paarweise in Ussing-Kammern befestigt und mit automatischen Spannungsklemmen (von der University of Iowa Bioengineering Department) kurzgeschlossen. Man lässt die Präparate anschliessend während 15 Minuten ins Gleichgewicht kommen. Die paarweise angeordneten Gewebeproben werden einerseits mit Bradykinin (oder Leukotrienen) und andererseits mit Bradykinin (oder Leukotrienen) und einer bestimmten Konzentration des Inhibitors behandelt. Man vergleicht dann die erhaltenen Antworten und berechnet daraus die Hemmung in Prozent.

Man verwendet in diesem Experiement Bradykinin, weil bekannt ist, dass diese Substanz die Phospholipase $A_2$ stimuliert, was zur Produktion von Leukotrienen führt, welche bekannterweise die Salzsekretion in intestinalen Gewebeproben in vitro fördern. Musch, et al., J. Clin. Invest. 71: 1073-1083 (1983); Hojvat, et

al., J. Pharmacol. Exp. Ther., 226: 749-755 (1983); Cuthbert, et al., Br. J. Pharmac. 82: 597-607 (1984): Musch, et al., Science 217: 1255-1256 (1982). Die Aktivität von Bradykinin bei der Produktion von Leukotrienen wird durch Inhibitoren der Leukotrien-Biosynthese unterdrückt; Cuthbert, et al., Br. J. Pharmac. 75: 587-589 (1982); Musch, et al., J. Clin. Invest., supra; Hojvat, et al., J. Pharmacol. Exp. Ther., supra.

Die erhaltenen Resultate sind in der Tabelle 1 und in der Figur 1 dargestellt. Diese zeigen die Fähigkeit der racemischen 6-Aceyl-7-[5-(4-acetyl-3-hydroxy-2-propylphenoxy)pentyloxy] -3,3-dihydro-2H-1-benzopyran-2-carbonsäure, die sekretorische Aktivität von Leukotrienen (Tabelle 1) und von Bradykinin (Figur 1) erfindungsgemäss zu reduzieren.

Tabelle 1

| Effekt des Inhibitors auf die Leukotrien-induzierte Elektrolyt-Sekretion durch das Kolon der Ratte (in vitro) | | | | |
|---|---|---|---|---|
| Inhibitor Konzentration in Mikromol (mM) | % Inhibition gegen Leukotriene* | | | |
| | $LTB_4$ | $LTC_4$ | $LTD_4$ | $LTE_4$ |
| 1 | 44,4±13,1 | 9,5±19,2 | 30,8±15,4 | 29,8±15,0 |
| 10 | 69,6±11,9 | 57,1 + 19,7 | 62,4±10,0 | 44,2± 9,8 |
| LT = Leukotrien | | | | |

* sämtliche in Prozent angegebenen Hemmwerte basieren auf Messungen an 6 Versuchstieren.

B. Antibiotika-induzierte Kolitis in Hamstern, in vivo

Man verabreicht männlichen, 80-120 g schweren syrischen Hamstern (LUG) eine einmalige Dosis von 175 mg/kg Clindamycin-phosphat oder Clindamycin-hydrochlorid intraperitoneal, um eine Kolitis zu induzieren. Ungefähr 7 Stunden nach dieser Injektion verabreicht man den Tieren die zu testende Verbindung oral oder intraperitoneal. Während einer Periode von 4 weiteren Tagen wird diese Therapie zweimal täglich wiederholt. Für die orale Verabreichung wurde die zu testende Verbindung in Wasser suspendiert oder in Dimethylsulfoxid gelöst und mittels einer Magensonde verabreicht. Der Effekt der Therapie wurde durch das sogenannte "Hazard"-Verhältnis bestimmt. Es handelt sich dabei um das Verhältnis der Sterblichkeitsrate von Tieren, welche mit der zu testenden Verbindung behandelt worden sind, im Vergleich zu den Tieren, welche nur mit Vehikel behandelt worden sind. Die Sterblichkeitsrate wurde zweimal täglich bestimmt und wurde durch Vergleich der Ueberlebenskurven für jede Gruppe ausgewertet. Für jede Gruppe von Versuchstieren wurde die Kaplan-Meier-Schätzung der Ueberlebenskurve berechnet, und es wurde der Mantel-Cox-Test verwendet, um die Ueberlebenskurve der mit Inhibitor behandelten Gruppe mit derjenigen der nur mit Vehikel behandelten Gruppe zu vergleichen. Ein Hazard-Verhältnis von 1,0 bedeutet, dass der Effekt der Therapie nicht besser ist, als derjenige der mit dem Vehikel allein erzielt wird, währenddem ein Hazard-Verhältnis (>1.0) bedeutet, das die Therapie die Ueberlebensrate im Vergleich zu derjenigen Gruppe, welche nur mit dem Vehikel behandelt worden ist, verbessert.

Es wurde gefunden, dass die racemische 6-Acetyl-7-[5-(4-acetyl-3-hydroxy-2-propylphenoxy)pentyloxy] -3,4-dihydro-2H-1-benzopyran-2-carbonsäure bei oralen Dosen im Bereich von 10 bis 100 mg/kg aktiv war. Zwei bekannte Leukotrien-Inhibitoren, nämlich 7-[-3-(4-Acetyl-3-hydroxy-2-propyl-phenoxy)-2-hydroxypropoxy] -4-oxo-8-propyl-4H-1-benzopyran-2-carbonsäure (vgl. Verbindung 1) und 7-[3-(4-Acetyl-3-hydroxy-2-propylphenoxy)propoxy]-4-oxo -8-propyl-4H-1-benzopryran-2-propancarbonsäure (vgl. Verbindung 2), erwiesen sich bei oralen Dosierungen von 10 mg/kg als inaktive (vgl. Tabelle 2).

Abgesehen davon, dass die racemische 6-Acetyl-7-[5-(4-acetyl-3-hydroxy-2-propylphenoxy)pentyloxy] -3,4-dihydro-2H-1-benzopyran-2-carbonsäure die Ueberlebensrate der mit Clidamycin behandelten Tiere erhöht, reduzierte diese Verbindung auch die Diarrhö und verbesserte in einem beträchtlichen Ausmass die Histopathologie des Cecums und des Kolons. Erwähnenswert ist insbesondere, dass des Cecum der mit Clindamycin behandelten Hamster anfänglich durch Oedeme, extensive Blutungen und Stauungen, Nekrose und fokale Erosionen der Schleimhäute, sowie durch eine erhöhte Infiltration der Schleimhäute durch Entzündungszellen charakterisiert war. Aehnliche, jedoch weniger schwerwiegende Wirkungen waren inbezug auf das Kolon zu verzeichnen. In den meisten Fällen wurden die Auswirkungen durch die Verabreichung von 60 oder 100 mg/kg der obigen Verbindung reduziert oder gänzlich zum Verschwinden gebracht.

## Tabelle 2

**Therapeutische Aktivität von Leukotrien-Inhibitoren gegen Antibiotika-induzierte Colitis (in vivo)**

| Testsubstanz | Dosis (mg/kg) | Hazardverhältnis | 95% Vertrauensbereich | n |
|---|---|---|---|---|
| racemische 6-Acetyl-7-[5- | 10[a] | 2,87 | 2,00- 4,13 | 20 |
| (4-acetyl-3-hydroxy-2- | 60[a] | 4,77 | 3,05- 7,45 | 20 |
| propylphenoxy)pentyloxy] | 10[b] | 3,80 | 2,64- 5,46 | 25 |
| 3,4-dihydro-2H-1-benzo- | 60[b] | 12,0 | 7,30-19,8 | 20 |
| pyran-2-carbonsäure | 100[b] | 64,2 | 22,5 -183 | 20 |
| Vergleichssubstanz 1 | 10[a] | 2,02[c] | 1,43- 2,85 | 20 |
| | 10[b] | 1,42 | 1,07- 1,89 | 25 |
| Vergleichssubstanz 2 | 13[b] | 1,68 | 1,25- 2,27 | 25 |

a = Intraperitoneale Verabreichung in 0,075 ml PEG-400
b = Orale Verabreichung in 0,15 ml Wasser
c = p <0,05 v. Vehikel durch Mantel-Cox und Global-Chi-Quadrat-Tests
d = p <0,01 v. Vehikel durch Mantel-Cox und Global-Chi-Quadrat-Tests
n = Anzahl Versuchstiere

Die racemische 6-Acetyl-7-[5-(4-acetyl-3-hydroxy-2-propylphenoxy)pentyloxy] -3,4-dihydro-2H-1-benzopryran-2-carbonsäure wurde zusammen mit dessen Methyl- und Hexylestern im Versuch mit der Clindamycin-induzierten Colitis in Hamstern in vivo nebeneinander evaluiert, und zwar wie oben beschrieben. Die Testsubstanzen wurden dabei in Dosierungen von 20 Mikromol/kg Körpergewicht oral verabreicht. Die mit den Estern erhaltenen Resultate sind in der nachfolgenden Tabelle 3 zusammengestellt. Der Vergleich mit der entsprechenden Carbonsäure, für welche eine Hazard-Verhältnis von 2,87 ermittelt worden ist (vgl. Tabelle 2), fällt dabei günstig aus

Tabelle 3

| Therapeutische Aktivität von Leukotrien-Inhibitoren gegen Antibiotika-induzierte Colitis (in vivo) | | | |
|---|---|---|---|
| Testsubstanz | Dosis (20 µMol/kg) | Hazardver- -hältnis | n |
| racemische 6-Acetyl-7-[5-(4-acetyl-3-hydroxy-2-propylphenoxy)-pentyloxy]-3,4-dihydro-2H-2-benzo-pyran-2-carbonsäure-methylester | 20 | 3,42 | 30 |
| racemische 6-Acetyl-7-[5-(4-acetyl-3-hydroxy-2-propylphenoxy)-pentyloxy]3,4-dihydro-2H-1-benzo-pyran-2-carbonsäure-methylester | 20 | 1,64 | 30 |
| racemische 6-Acetyl-7-[5-(4-acetyl-3-hydroxy-2-propylphenoxy)-pentyloxy]-3,4-dihydro-2H-1-benzo-pyran-2-carbonsäure | 20 | 1,45 | 30 |
| n = Anzahl Versuchstiere | | | |

C. Essigsäure-induzierte Colitis in Ratten, in vivo

Der Essigsäure-induzierte-Colitis-Bioassay an Ratten ist durch J.E. Krawisz et al. in Amer. J. Proc. Gastro. Col. Rec. Surg. 31, 11-18 (1980) und P. Sharon und W.F. Stenson in Gastroenterology 88, 55-63 (1985) und 86, 453-460 (1984) beschrieben worden. Die Essigsäure-induzierte Colitis ist durch eine Wanderung von Entzündungszellen in das Kolon charakterisiert. Die Anzahl derartiger Zellen in der Mucosa kann bestimmt werden, indem man die Aktivität der Myeloperoxidase, einem Marker-Enzym für diese Zellen, misst. Eine positive gewünschte Wirkung zeigt sich in einer Reduktion des hohen Myeloperoxidase-Spiegels, der durch die Essigsäure verursacht wird. Männliche, 150-300 g schwere Ratten (Sprague-Dawley) werden während 2 Tagen, zweimal täglich entweder mit dem Vehikel (Wasser oder Dimethylsulfoxid) oder der im Wasser suspendierten oder in Dimethylsulfoxid gelösten Testsubstanz oral vorbehandelt. Am 3. Tag behandelt man die Tiere noch einmal wie an den beiden Vortagen, anästhetisiert mit Metofan, injiziert 2 ml 2,5-proz. Essigsäure mittels einer Spritze in das Lumen des Kolons und unmittelbar danach 3 ml Luft und eine Spüllösung, bestehend aus 3 ml Phosphat-gepufferter Kochsalzlösung, bestehend bleibt genügend lange im Lumen, um eine Entzündung zu induzieren, ohne jedoch schwere Nekrose oder irreversible Schäden zu bewirken). Ungefähr 16 Stunden später verabreicht man den Tieren eine weitere Dosis der Testsubstanz, und zwar in der gleichen Menge. 24 Stunden nach der Behandlung mit der Essigsäure werden die Tiere getötet, worauf man die Mucosa des Kolons operativ entfernt und in einem wässrigen Puffer bei pH mit einem geeigneten Mixer homogenisiert. Die Aktivität der Myeloperoxidase im Homogenat wird unter Verwendung von o-Phenylendiamin als Chromogen, wie durch A. Voller, D.E. Bidwell und A. Bartlett in The Enzyme Linked Immunosorbent Assay (ELISA), Zoological Soc., London, 1979, Seiten 29-30 beschrieben, gemessen. Kontrolltiere werden mit dem Vehikel vorbehandelt und anschliessend mit Kochsalzlösung anstelle der Essigsäure behandelt.

Unter Verwendung des oben beschriebenen Testes wurde die racemische 6-Acetyl-7-[5-(4-acetyl-3-hydroxy-2-propylphenoxy)pentyloxy] -3,4-dihydro-2H-1-benzopyran-2-carbonsäure mit Phenidon verglichen, einem bekannten Inhibitor der Leukotrien-Synthese. Man erhielt die in den Figuren 2 und 3 gezeigten Resultate. Wie man sieht, war Phenidon in oralen Dosierungen von 100 und 200 mg/kg (Figur 2) aktiv, währenddem die racemische 6-Acetyl-7-[5-(4-acetyl-3-hydroxy-2-propylphenoxy)pentyloxy] -3,4-dihydro-2H-1-benzopyran-2-carbonsäure sogar bei den kleineren oralen Dosen von 30 und 90 mg/kg (Figur 3) aktiv war.

D. Carrageenin-induzierte Colitis in Meerschweinchen, in vivo

Der Carrageenin-induzierte-Colitis-Bioassay an Meerschweinchen ist durch A. B. Onderdonk in Inflammatory Bowel Diseases, Martinus Nijhoff Publ., Boston, Massachusetts, Seiten 126-134 (1982) und in Human Intestinal Microflora in Health and Disease (D. J. Hentger. ed), Academic Press, New York, 1983, Seiten 447-479 beschrieben worden. Das aus Rotalgen herstammende Polysaccharid Jotacarrageenin bewirkt in Meerschweinchen eine Colitis, welche der ulcerativen Colitis im Menschen ähnlich ist. Männliche und weibliche, 400-650 g Meerschweinchen (Hartley) erhielten via Trinkwasser täglich eine 3-proz. Lösung von abgebautem Carrageenin. Nach etwa 7 Tagen begannen die Versuchstiere eine Diarrhö zu entwickeln, nahmen an Gewicht nicht mehr zu und wurden lethargisch. Nachdem die Versuchstiere während 14 Tagen auf diese Weise Carrageenin verabreicht erhielten, wurde die Diarrhö quantifiziert, und zwar durch Messung des Wassertransportes in den distalen Teil des Kolons durch einfache Pass-Perfusion unter Verwendung von 14C-Polyäthylenglykol 4000 (PEG 4000) als Marker. Kontrolltiere erhielten eine wässrige Trinklösung, enthaltend 3-proz. mannitol anstelle des Carrageenins. Gleichzeitig wurden zufällig ausgewählte Versuchstiere aus diesen beiden Gruppen zweimal täglich mit der Testsubstanz oral behandelt.

Die racemische 6-Acetyl-7-[5-(4-acetyl-3-hydroxy-2-propylphenoxy)pentyloxy] -3,4-dihydro-2H-1-benzopyran-2-carbonsäure wurde in diesem Test mit dem Sulfasalazin, einem Standard-Inhibitor in diesem Testsystem, verglichen. Die Resultate sind in den Figuren 4 und 5 dargestellt.

Wie man sehen kann, bewirkt das Sulfasalazin bei einer oralen Dosis von 100 mg/kg die Wiederherstellung des Wassertransportes im Kolon der kranken Versuchstiere von einem sekretorischen Niveau auf ein Niveau, das ungefähr demjenigen der Kontrol-Versuchstiere (Figur 4) entspricht. Andererseits zeigten Versuchstiere, welche mit racemischer 6-Acetyl-7-[5-(4-acetyl-3-hydroxy-2-propylphenoxy)pentyloxy] -3,4-dihydro-2H-1-benzopyran-2-carbonsäure behandelt worden sind, und zwar bei niedrigeren Dosierungen von 10 bzw. 30 mg/kg, Wassertransport-Raten, welche praktisch gleich waren, wie diejenigen der Kontroll-Versuchstiere (Figur 5).

Beispiel 1

| Orale Formulierung (Tablette) | |
|---|---|
| Bestandteile | mg/Tablette |
| Racemische 6-Acetyl-7-[5-(4-acetyl-3-hydroxy-2-propylphenoxy)pentyloxy]-3,4-dihydro-2H-1-benzopyran-2-carbonsäure | 250.0 mg |
| Lactose | 50.0 mg |
| Stärke | 25.0 mg |
| Polyvinylpyrrolidon | 2.5 mg |
| Magnesiumstearat | 2.5 mg |
| Gesamtgewicht | 330.0 mg |

Verfahren:

Man granuliert eine Mischung aus den pulverisierten Bestandteilen mit dem Polyvinylpyrrolidon in Lösung und verpresst zu Tabletten. Anschliessend werden die Tabletten überzogen, indem man sie mit der nachfolgenden beschriebenen Lösung besprüht oder sie in einem Bad, das diese Lösung enthält, rotiert:

| | Gew.-% |
|---|---|
| Hydroxypropylmethylcellulosephthalat<br>Alcohol 3A<br>Methylenchlorid | 6<br>47<br>47 |
| Gesamtgewicht | 100% |

Die erhaltenen lackierten Tabletten sind gegenüber dem Magensaft resistent, lösen sich jedoch im Darmsaft auf, wenn man sie im in-vitro-Auflösungstest gemäss USP untersucht.

Beispiel 2

| Orale Formulierung (Weichgelatinekapsel) | |
|---|---|
| Bestandteile | mg/Kapsel |
| Natriumsalz von racemischer 6-Acetyl-7-[5-(4-acetyl-3-hydroxy-2-propylphenoxy)-pentyloxy]-3,4-dihydro-2H-1-benzopyran-2-carbonsäure | 261.0* mg |
| Triglyceride (mittlere Kettenlänge) | 229.0 mg |
| Natriumsilikat | 10.0 mg |
| Gesamtgewicht | 500.00 mg |

* entspricht 250 mg der freien Säure

Verfahren:

Die Bestandteile werden in Form einer Suspension zubereitet, welche dann in Weichgelatinekapseln abgefüllt wird. Die Kapseln werden anschliessend mit einem geeigneten Lackiermittel überzogen, damit sie gegenüber dem Magensaft resistent sind, sich jedoch im Darmsaft auflösen, wenn man sie im in vitro-Auflösungstest gemäss USP untersucht.

Beispiel 3

| Orale Formulierung (Mikrokapseln) | |
|---|---|
| Bestandteile | mg/Dosis |
| racemische 6-Acetyl-7-[5-(4-acetyl-3-hydroxy-2-propylphenoxy)pentyloxy]-3,4-dihydro-2H-1-benzopyran-2-carbonsäure | 250 mg |
| Polyvinylpyrrolidon | 25 mg |
| Stärke | 50 mg |
| Gesamtgewicht/Dosis | 325 mg |

Verfahren:

Die Mikrokapseln werden durch feuchte Granulierung hergestellt und dann mit der polymeren Lösung nachfolgender Zusammensetzung lackiert, indem man die Oberfläche der Mikrokapsel in einem rotierenden, geeigneten Behältnis besprüht.

| | Gew.-% |
|---|---|
| Polymethacrylsäure-Polymer | 5.0 |
| Aethylcellulose | 1.0 |
| Aceton | 10.0 |
| Isopropylalkohol | 84.0 |
| Gesamtgewicht | 100.0 % |

Die lackierten Mikrokapseln sind gegenüber dem Darmsaft resistent, lösen sich jedoch im Darmsaft auf, wenn man sie im in vitro-Auflösungstest gemäss USP untersucht.

Beispiel 4

| Orale Formulierung (Tablette) | | |
| --- | --- | --- |
| Bestandteile | mg/Tablette | |
| | 100 mg | 600 mg |
| racemische 6-Acetyl-7-[5-(4-acetyl-3-hydroxy-2-propylphenoxy)pentyloxy]-3,4-dihydro-2H-1-benzopyran-2-carbon-säure | 100 mg | 600 mg |
| Lactose | 30 mg | 100 mg |
| Vorgelatinisierte Stärke | 4 mg | 25 mg |
| Mikrokristalline Cellulose | 20 mg | 120 mg |
| Modifizierte Stärke | 5 mg | 25 mg |
| Magnesiumstearat | 1 mg | 8 mg |
| Gesamtgewicht | 160 mg | 878 mg |

Verfahren:

Die racemische 6-Acetyl-7-[5(4-acetyl-3-hydroxy-2--propylphenoxy)pentyloxy] -3,4-dihydro-2H-1-benzopyran-2--carbonsäure, die Lactose, die vorgelatinisiert Stärke und die mikrokristalline Cellulose werden vermischt und im Wasser granuliert. Das granulierte Material wird bei 45-50 °C getrocknet und vermahlen. Die modifizierte Stärke und das Magnesiumstearat werden dann dazugegeben, und die erhaltene Mischung wird zu Tabletten verpresst.

Beispiel 5

Orale Formulierung (Tablette)

| Bestandteile | mg/Tablette | |
|---|---|---|
| | 100 mg | 600 mg |
| racemische 6-Acetyl-7-[5-(4-acetyl-3-hydroxy-2-propylphenoxy)pentyloxy]-3,4-dihydro-2H-1-benzopyran-2-carbonsäure | 100 mg | 600 mg |
| Lactose | 40 mg | 200 mg |
| Polyvinylpyrrolidon | 4 mg | 24 mg |
| modifizierte Stärke | 4 mg | 24 mg |
| Magnesiumstearat | 1 mg | 6 mg |
| Gesamtgewicht | 149 mg | 856 mg |

Verfahren:

Man vermischt die racemische 6-Acetyl-7-[5-(4-acetyl-3--hydroxy-2-propylphenoxy)pentyloxy] -3,4-dihydro-2H-1-benzopyran-2-carbonsäure, die Lactose und die modifizierte Stärke, granuliert mit Polyvinylpyrrolidon in Wasser oder Alkohol, trocknet das granulierte Material bei 45-50 °C, vermahlt, gibt das Magnesiumstearat dazu und verpresst das erhaltene Gemisch zu Tabletten.

Beispiel 6

| Orale Formulierung (Kapseln) | | |
|---|---|---|
| Bestandteile | mg/Kapsel | |
| | 100 mg | 600 mg |
| racemische 6-Acetyl-7-[5-(4-acetyl-3-hydroxy-2-propylphenoxy)pentyloxy]-3,4-dihydro-2H-l-benzopyran-2-carbonsäure | 100 mg | 600 mg |
| Lactose* | 50 mg | -- |
| vorgelatinisierte Stärke** | 5 mg | 30 mg |
| Talk | 4 mg | 24 mg |
| Magnesiumstearat | 1 mg | 6 mg |
| Gesamtgewicht | 160 mg | 660 mg |

* kann durch Dicalciumphosphat-dihydrat, Mannitol oder Sorbitol ersetzt werden

** kann durch Hydroxypropylmethylcellulose, Gelatine oder Silikate ersetzt werden

Verfahren:

Man vermischt die racemische 6-Acetyl-7-[5-(4-acetyl-3-hydroxy-2-propylphenoxy)pentyloxy] -3,4-dihydro-2H-1-benzopyran-2-carbonsäure, die Lactose und die vorgelatinisierte Stärke, granuliert in Wasser, trocknet das granulierte Material bei 45-50° C, vermahlt, gibt den Talk und das Magnesiumstearat dazu und füllt die erhaltene Mischung in Weichgelatinekapseln ab.

Beispiel 7

| Parenterale Formulierung | |
|---|---|
| Bestandteile | mg/ml |
| racemische 6-Acetyl-7-[5-(4-acetyl-3-hydroxy-2-propylphenoxy)pentyloxy]-3,4-dihydro-2H-1-benzopyran-2-carbonsäure | 10 mg |
| Mannitol* | 20 mg |
| Natriumhydroxid und/oder Salzsäure zum justieren des pH | q.s. |
| Wasser (für Injektion), s.q. ad | 1.0 ml |
| Gesamtvolumen | 1.0 ml |

* kann durch Lactose ersetzt werden

Verfahren:

Man löst die racemische 6-Acetyl-7-[5(4-acetyl-3-hydroxy-2-propylphenoxy)pentyloxy] -3,4-dihydro-2H-1-benzopyran-2-carbonsäure und das Mannitol in Wasser und justiert den pH. Die Lösung wird anschliessend in einem geeigneten Behältnis lyophilisiert (Entfernen des Wassers).

Beispiel 8

Parenterale Formulierung

| Bestandteile | Gew.-Prozent |
|---|---|
| racemische 6-Acetyl-7-[5-(4-acetyl-3-hydoxy-2-propylphenoxy)pentyloxy]-3,4-dihydro-2H-1-benzopyran-2-carbonsäure | 1.0% |
| Benzylalkohol* | 1.0% |
| Propylenglykol** | 10.0% |
| Emulphor** | 5.0% |
| Phosphatpuffer, s.q. pH | 7.5 |
| Wasser (für Injektion), s.q. ad | 100% |

* kann durch Thimerosal, Phenol, Methyl und Propylparaben, Benzothoniumchlorid oder Chlorbutanol ersetzt werden

** kann durch Polyäthylenglykol, Alkohol, Dimethylacetamid, Glycerin, Povidon, Lecithin, Sorbitan-monooleat und -trioleat ersetzt werden

Verfahren:

Man löst den Phosphatpuffer in einem Teil des Wassers, gibt den Benzylalkohol, das Propylenglykol und Emulphor unter Rühren in die gepufferte Lösung dazu, löst dann die racemische 6-Acetyl-7-[5-(4-acetyl-3-hydroxy-2-propyl-phenoxy)pentyloxy] -3,4-dihydro-2H-1-benzopyran-2-carbonsäure in der Lösung auf, justiert den pH, gibt Wasser bis zur gewünschten Menge dazu und füllt die erhaltene Lösung in ein geeignetes Behältnis ab.

Beispiel 9

| Rektale Formulierung (Suppositorien) | | |
|---|---|---|
| Bestandteile | mg/Suppositorium | |
| | 100mg | 1000mg |
| racemische 6-Acetyl-7-[5-(4-acetyl-3-hydroxy-2-propylphenoxy)pentyloxy]-3,4-dihydro-2H-1-benzopyran-2-carbonsäure | 100 mg | 1000 mg |
| Suppositoriummasse*, s.q. ad | 1000 mg | 2000 mg |
| Gesamtgewicht | 1000 mg | 2000 mg |

* enthält semi-synthetische Glyceride, Mischungen von Triglyceriden, wie Witepsol H15 der Dynamit Nobel Chemicals, und die Polyäthylenglykole 400 und 4000, einzeln oder gewünschtenfalls in Kombination

Verfahren:

Man dispergiert die racemische 6-Acetyl-7-[5-(4-acetyl--3-hydroxy-2-propylphenoxy)pentyloxy] -3,4-dihydro-2H-1-benzopyran-2-carbonsäure in der geschmolzenen Suppositorienmasse und füllt die erhaltene Masse in geeignete Suppositorienformen.

Beispiel 10

| Rektale Formulierung (Suppositorien) | | |
|---|---|---|
| Bestandteile | mg/Suppositorium | |
| | 100 mg | 1000 mg |
| racemische 6-Acetyl-7-[5-(4-acetyl-3-hydroxy-2-propylphenoxy)pentyloxy]-3,4-dihydro-2H-1-benzopyran-2-carbonsäure | 100 mg | 1000 mg |
| Sorbitantrioleat | 100 mg | 600 mg |
| Kakaobutter, s.q. ad | 1000 mg | 2000 mg |
| Gesamtgewicht | 1000 mg | 2000 mg |

Verfahren:

Man schmilzt die Kakaobutter, dispergiert das Natriumsalz der racemischen 6-Acetyl-7-[5-(4-acetyl-3-hydroxy-2-propyl-phenoxy)pentyloxy] -3,4-dihydro-2H-1-benzopyran-2-carbonsäure und das Sorbitantrioleat darin und füllt die Masse in geeignete Suppositorienformen.

Beispiel 11

| Rektale Formulierung (Klistier) | | |
|---|---|---|
| Bestandteile | mg/Dosis | |
| | 100 mg | 1000 mg |
| Natriumsalz von racemischer 6-Acetyl-7-[5(4-acetyl-3-hydroxy-2-propylphenoxy)pentyloxy]-3,4-dihydro-2H-1-benzopyran-2-carbonsäure | 100 mg | 1000 mg |
| Polyäthylenglykol 400 | 10 ml | 15 ml |
| Wasser für Injektion, s.q. ad | 120 ml | 180 ml |
| Gesamtvolumen/Dosis | 120 ml | 180 ml |

Verfahren:

Das Natriumsalz der racemischen 6-Acetyl-7-[5-(4-acetyl-3-hydroxy-2-propylphenoxy)pentyloxy] -3,4-dihydro-2H-1-benzopyran-2-carbonsäure und das Polyäthylenglykol 400 werden vorgemischt und dann in Wasser für Injektionszwecke gelöst. Diese Lösung wird dann in geeignete Behälter abgefüllt aus denen es dann durch Drücken dispensiert werden kann.

Beispiel 12

In Analogie zu den Beispielen 1-11 können Tabletten, Kapseln, Mikrokapseln, parenterale und rektale Formulierungen hergestellt werden, welche die nachfolgenden Verbindungen enthalten:

Racemische 6-Acetyl-7-[5-(4-acetyl-3-hydroxy-2-propylphenoxy)pentyloxy]-3,4-dihydro-2H-1-benzopyran-2-carbonsäuremethylester und

racemische 6-Acetyl-7-[5-(4-acetyl-3-hydroxy-2-propylphenoxy)pentyloxy]-3,4-dihydro-2H-1-benzopyran-2-carbonsäurehexylester.

Die nachfolgenden Beispiele 13-15 beschreiben die Herstellung der noch neuen Methyl- und Hexylester der racemischen 6-Acetyl-7-[5-(4-acetyl-3-hydroxy-2-propylphenoxy)pentyloxy] -3,4-dihydro-2H-1-benzopyran-2-carbonsäure.

Beispiel 13

Herstellung des racemischen 6-Acetyl-7-[5-(4-acetyl-3-hydroxy-2-propylphenoxy)pentyloxy]-3,4-dihydro-2H-1-benzopyran-2-carbonsäure-methylester

Eine Mischung aus 2 g racemischer 6-Acetyl-7-[5-(4-acetyl-3-hydroxy-2-propylphenoxy)pentyloxy] -3,4-dihydro-2H-1-benzopyran-2-carbonsäure, 97 mg p-Toluolsulfonsäure-monohydrat und 25 ml Methanol wird unter Rühren während 19 Stunden unter Rückfluss zum Sieden erhitzt. Die erhaltene Lösung wird abgekühlt und im Vakuum eingedampft, wobei man einen festen Rückstand erhält, den man in Essigester aufnimmt. Die Essigesterlösung wird mit gesättigter wässriger Natriumbicarbonatlösung, Wasser und gesättigter Kochssalzlösung gewaschen und anschliessend über wasserfreiem Magnesiumsulfat getrocknet, filtriert und im Vakuum eingedampft. Der Rückstand wird an 100 g Kieselgel chromatographiert. Nach Eluieren mit einer 1:1-Mischung von Hexan und Essigester erhält man 2,06 g des reinen Methylesters. Durch Umkristallisieren aus Acetonitril erhält man den racemischen 6-Acetyl-7-[5-(4-acetyl-3-hydroxy-2-propylphenoxy)-pentyloxy] -3,4-dihydro-2H-1-benzopyran-2-carbonsäure-methylester als farblosen, amorphen Festkörper.

| | | |
|---|---|---|
| Analyse berechnet für $C_{29}H_{36}O_8$: | C, 67,96%; | H, 7,08%. |
| Gefunden: | C, 67,69%; | H, 6,98%. |

Beispiel 14

Herstellung des racemischen 6-Acetyl-7-[5-(4-acetyl-3-hydroxy-2-propylphenoxy)pentyloxy]-3,4-dihydro-2H-1-benzopyran-2-carbonsäure-methylester

Eine Mischung aus 20,2 g racemischen 6-Acetyl-7-[5-(brompentyloxy]-3,4-dihydro-2H-1-benzopyran -2-carbonsäuremethylester, 11,0 g 2,4-Dihydroxy-3-propylacetophenon, 25,4 g wasserfreiem Kaliumcarbonat, 436 ml trockenem Aceton und 218 ml trockenem N,N-Dimethylformamid wird während 5,5 Stunden unter Rühren und unter Rückfluss zum Sieden erhitzt. Das Reaktionsgemisch wird abgekühlt und mit Essigester verdünnt. Die organische Phase wird mit Wasser und gesättigter Kochsalzlösung gewaschen, anschliessend über Magnesiumsulfat getrocknet, filtriert und im Vakuum eingedampft. Man erhält 28,3 g eines bernsteinfarbenen Oeles. Dieses Material wird in einem 1:1-Gemisch von Hexan und Essigester gelöst und mittels präparativer HPLC (Waters 500 A, 2 Kolonnen) unter Verwendung von Hexan/Essigester (1:1) als Elusionsmittel gereinigt. Man erhält 24,83 g (96,8%) des gewünschten Esters als Oel, das beim Stehenlassen zu einem farblosen Festkörper kristallisiert.

Beispiel 15

Herstellung des racemischen 6-Acetyl-7-[5-(4-acetyl-3-hydroxy-2-propylphenoxy)pentyloxy]-3,4-dihydro-2H-1-benzopyran-2-carbonsäure-hexylesters

In einem 1-1 Kolben, der mit einem mechanischen Rührer, einem Dean-Stark-Apparat, einem Rückflusskühler umd einem Calciumchloridrohr ausgerüstet ist, werden 50,0 g racemische 6-Acetyl-7-[5-(4-acetyl-3-hydroxy-2-propylphenoxy)pentyloxy]-3,4-dihydro-2H-1-benzopyran-2-carbonsäure, 10,3 g 1-Hexanol, 1,0 g p-Toluolsulfonsäure-monohydrat und 500 ml Toluol vorgelegt. Die Mischung wird anschliessend unter einer Inertgasatmosphäre während 2 Stunden zum Sieden erhitzt, wobei das entstehende Reaktionswasser laufend entfernt wird. Nach dem Abkühlen auf Raumtemperatur wird die erhaltene braune Lösung nacheinander zweimal mit je 100 ml gesättigter Kochsalzlösung, zweimal mit je 100 gesättigter wässeriger Natriumbicarbonatlösung und zweimal mit je 100 ml gesättigter Kochsalzlösung gewaschen. Die organische Phase wird über wasserfreiem Natriumsulfat getrocknet, filtriert und unter vermindertem Druck zunächst bei 4,55 kPa und schliesslich im Hochvakuum bei 13 Pa und bei 45 ° C zur Trockene eingedampft, wobei man ein braunes Oel erhält. Dieses Oel wird bei Raumtemperatur bis zur beginnenden Kristallisation mit 370 ml Alkohol behandelt, worauf man die Mischung über Nacht bei 12 ° C in den Kühlschrank stellt. Die Kristalle werden dann abfiltriert und über Nacht an der Luft getrocknet. Man erhält 50,4 g (86%) racemischen 6-Acetyl-7-[5-(4-acetyl-3-hydroxy-2-propylphenoxy)pentyloxy] -3,4-dihydro-2H-1-benzopryran-2-carbonsäure-hexylester als weisslichen Festkörper vom Schmelzpunkt 70-72 ° C.

**Patentansprüche**

1. Verwendung einer Verbindung der allgemeinen Formel

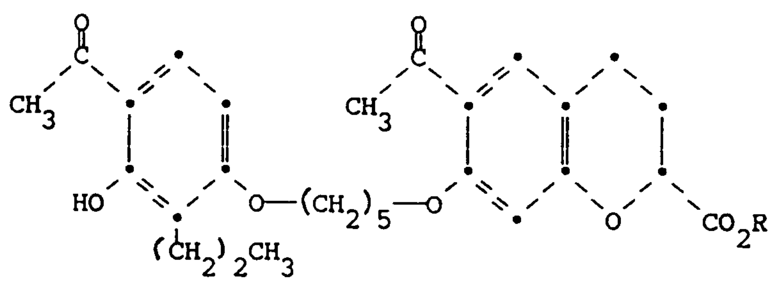

I

worin R Wasserstoff oder $C_1$-$C_7$-Alkyl bedeutet,
eines Enantiomeren davon oder, falls R Wasserstoff bedeutet, eines pharmazeutisch annehmbaren Salzes davon mit einer Base zur Herstellung von pharmazeutischen Präparaten gegen Enteritis und anderen durch Leukotrien vermittelten Entzündungen der intestinalen Mucosa.

2. Die Verwendung von racemischer 6-Acetyl-7-[5-(4-acetyl-3-hydroxy-2-propylphenoxy)pentyloxy] -3,4-dihydro-2H-1-benzopyran-2-carbonsäure gemäss Anspruch 1.

## Claims

**1.** The use of a compound of the general formula

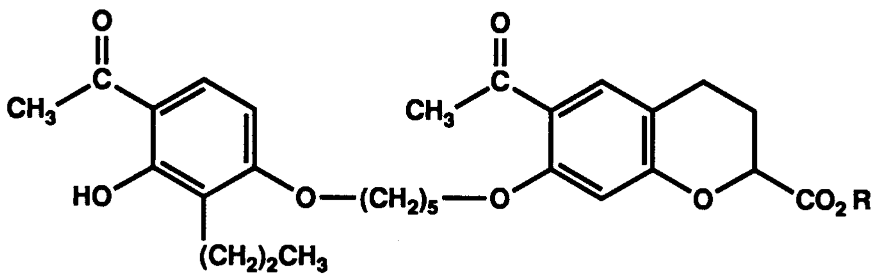

I

wherein R signifies hydrogen or $C_1$-$C_7$ alkyl,
of an enantiomer thereof or, where R signifies hydrogen, of a pharmaceutically acceptable salt thereof with a base for the manufacture of pharmaceutical preparations against enteritis and other leukotriene-mediated inflammations of the intestinal mucosa.

**2.** The use of racemic 6-acetyl-7-[5-(4-acetyl-3-hydroxy-2-propylphenoxy)pentyloxy]-3,4-dihydro-2H-1-benzopyran-2-carboxylic acid in accordance with claim 1.

## Revendications

**1.** Application d'un composé de formule générale

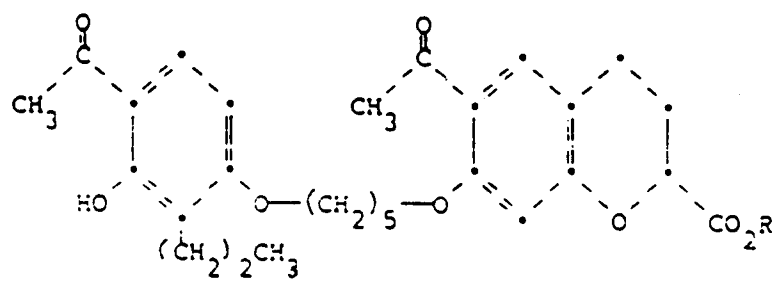

I

dans laquelle R représente un hydrogène ou un alcoyle en $C_1$ à $C_7$ , d'un de ses énantiomères ou, si R représente un hydrogène, d'un de ses sels pharmaceutiquement acceptable avec une base, à la préparation de préparations pharmaceutiques contre l'entérite et d'autres inflammations de la muqueuse intestinale provoquées par le leucotriène.

**2.** Application du racémique de l'acide 6-acétyl-7-[5-(4-acétyl-3-hydroxy-2-propylphénoxy)pentyloxy]-3,4-dihydro-2H-1-benzopyranne-2-carboxylique selon la revendication 1.

FIGUR 1

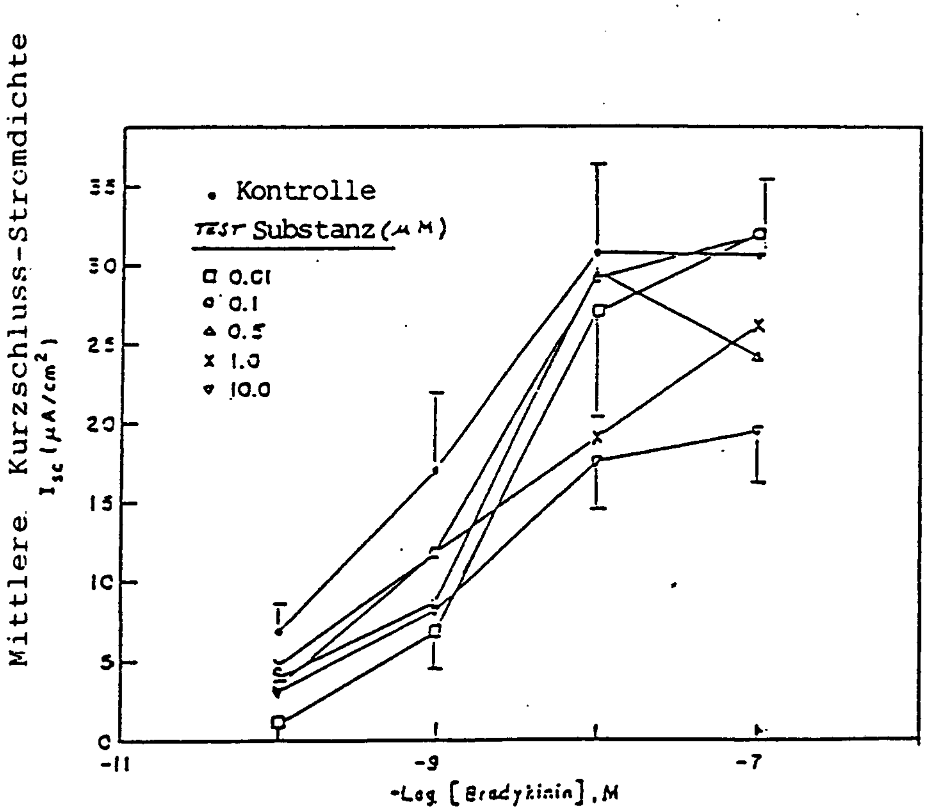
Mittlere Kurzschluss-Stromdichte
$I_{sc}$ ($\mu A/cm^2$)
• Kontrolle
TEST Substanz ($\mu M$)
0.01
0.1
0.5
1.0
10.0
-Log [Bradykinin], M

FIGUR 2

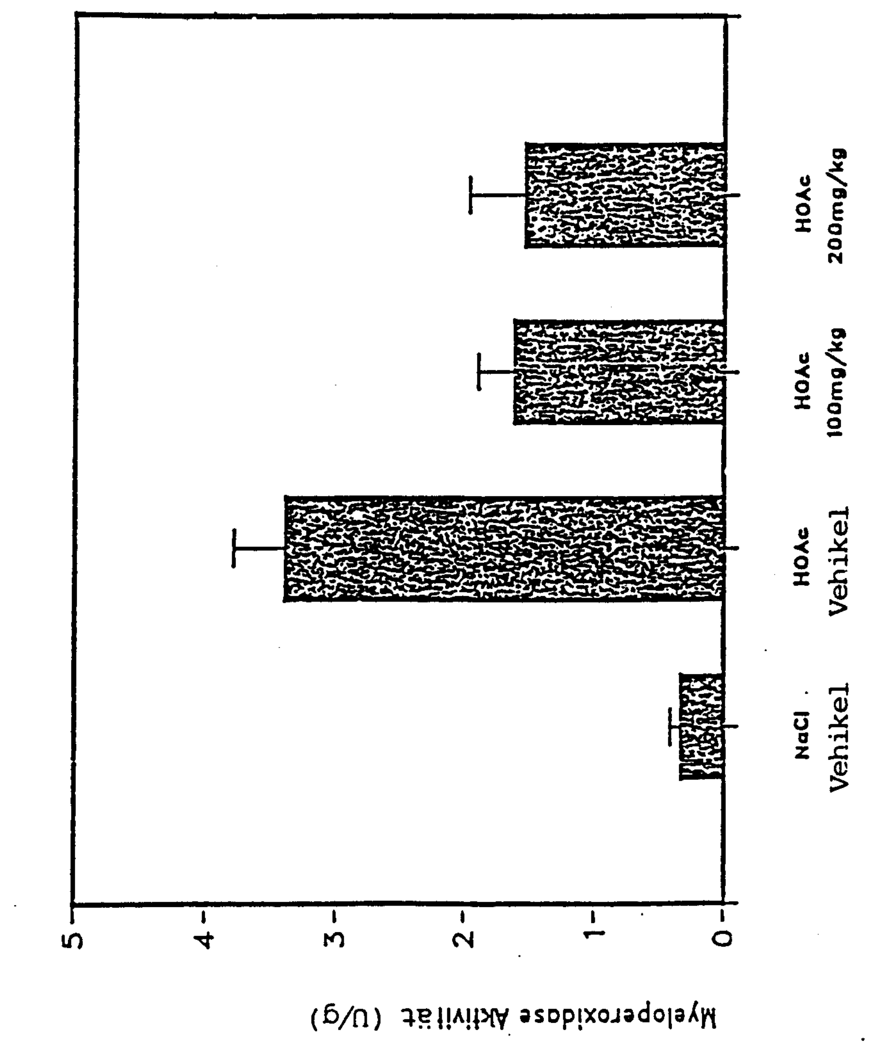
Myeloperoxidase Aktivität (U/g)
5
4
3
2
1
0
NaCl
Vehikel
HOAc
Vehikel
HOAc
100mg/kg
HOAc
200mg/kg

FIGUR 3

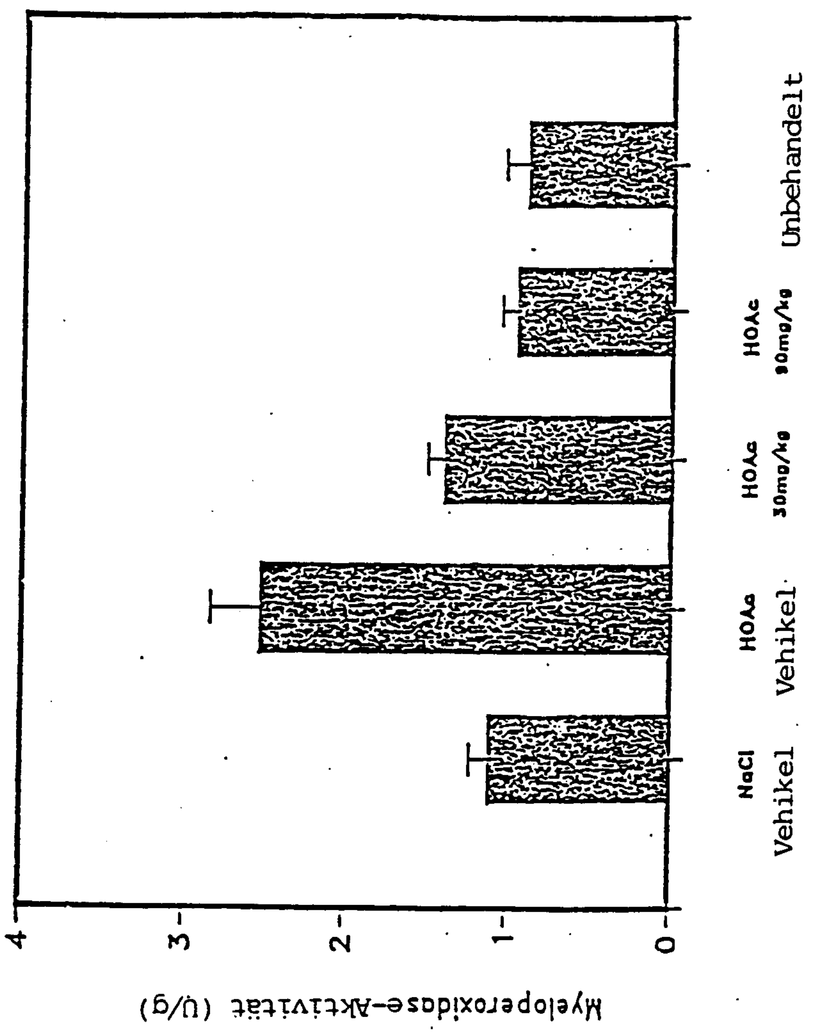
Myeloperoxidase-Aktivität (U/g)
0
1
2
3
4
NaCl
Vehikel
HOAc
Vehikel
HOAc
30mg/kg
HOAc
90mg/kg
Unbehandelt

FIGUR 4

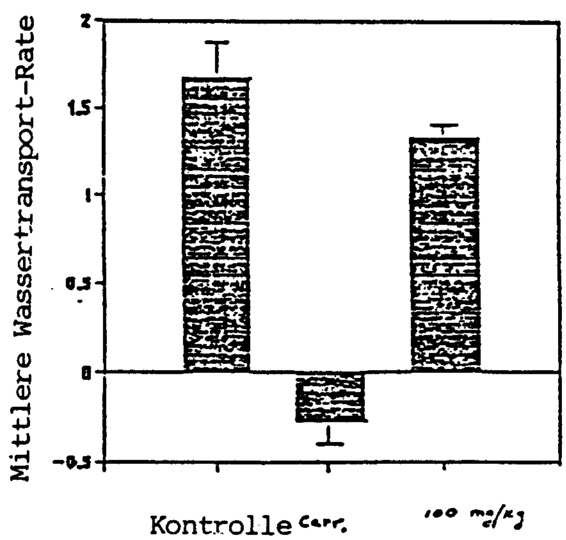
Mittlere Wassertransport-Rate
2
1.5
1
0.5
0
-0.5
Kontrolle
Carr.
100 mg/kg

FIGUR 5

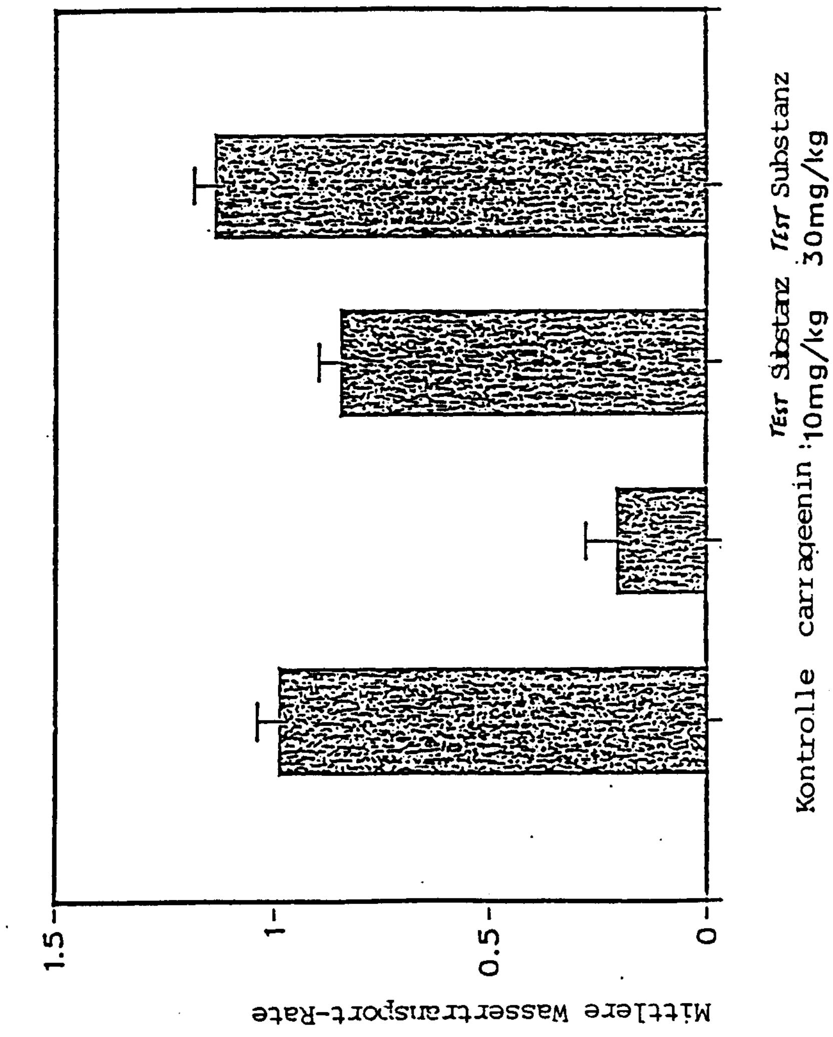
Mittlere Wassertransport-Rate
1.5
1
0.5
0
Kontrolle
carrageenin
Test Substanz 10mg/kg
Test Substanz 30mg/kg